# FASCICULE DE BREVET EUROPEEN

(11) **EP 0 701 430 B1**
(45) Date de publication et mention de la délivrance du brevet: **23.04.1997**
(21) Numéro de dépôt: 94917692.9
(22) Date de dépôt: 31.05.1994
(51) Int. Cl.: A61K 7/50, A61K 7/48, A61K 7/06

(54) **COMPOSITIONS COSMETIQUES CONTENANT AU MOINS UN TENSIOACTIF ANIONIQUE DU TYPE ALKYLGALACTOSIDE URONATE ET AU MOINS UN POLYMERE AMPHOTERE**
KOSMETISCHE MITTEL ENTHALTEND MINDESTENS EIN ANIONISCHES TENSID VOM TYP ALKYLGALAKTOSID-URONAT UND MINDESTENS EINEN AMPHOTOREN POLYMER
COSMETIC COMPOUNDS CONTAINING AT LEAST ONE ANIONIC ALKYLGALACTOSIDE URONATE TYPE SURFACE-ACTIVE AGENT AND AT LEAST ONE AMPHOTERIC POLYMER

(30) Priorité: 01.06.1993 FR 9306530
(43) Date de publication de la demande: 20.03.1996
(73) Titulaire: L'OREAL, 75008 Paris (FR)
(72) Inventeur: CAUWET, Danièle, F-75011 Paris (FR); DUBIEF, Claude, F-78150 Le Chesnay (FR)
(74) Mandataire: Casalonga, Axel
(86) Numéro de dépôt international: FR9400631
(87) Numéro de publication internationale: WO9427573

(56) Documents cités:
- EP-A- 0 532 370

## Description

L'invention concerne des compositions cosmétiques contenant au moins un tensio-actif anionique du type alkylgalactoside uronate et au moins un polymère amphotère, ainsi que des procédés de traitements cosmétiques des matières kératiniques mettant en oeuvre de telles compositions.

Les compositions de lavage des cheveux ou de la peau sont généralement formulées à partir d'agents tensio-actifs anioniques ou non-ioniques ou leurs mélanges en présence éventuellement d'agents tensio-actifs amphotères.

Les cheveux agressés par les agents atmosphériques tels que la lumière ou les traitements chimiques et lavés avec les compositions de lavage classiques sont difficilement démêlables et cet inconvénient se trouve encore accentué dans le cas de cheveux fins.

Les compositions de lavage des cheveux utilisant ces tensio-actifs anioniques ou non-ioniques seuls ne conduisent pas à de bonnes propriétés cosmétiques en particulier le démêlage des cheveux mouillés est difficile, et la mise en forme et la tenue de la coiffure dans le temps ne sont pas satisfaisants.

Les tensio-actifs anioniques du type alkylgalactoside uronate ont déjà été préconisés dans des compositions lavantes pour les cheveux. Ils ont été décrits dans la demande de brevet EP 0 532.370.

La demanderesse vient de découvrir de manière surprenante, que l'association, dans des compositions lavantes et/ou traitantes pour matières kératiniques, d'un tensio-actif anionique du type alkylgalactoside uronate et d'un polymère amphotère confère à ces compositions des propriétés de démêlage améliorées, en particulier des cheveux mouillés.

En outre, cette association confère aux cheveux séchés de la tenue et du gonflant, ainsi que de la nervosité aux cheveux bouclés.

La présente invention a donc pour objet des compositions cosmétiques contenant au moins un agent tensio-actif anionique du type alkylgalactoside uronate et au moins un polymère amphotère.

Un autre objet de l'invention est constitué par l'utilisation de ces compositions pour le traitement et/ou le lavage des matières kératiniques telles que les cheveux ou la peau.

Un autre objet concerne des procédés de traitements cosmétiques des cheveux ou de la peau au moyen des compositions de l'invention; les procédés de lavage et de traitement des cheveux étant préférés.

Les compositions cosmétiques selon l'invention contiennent dans un milieu aqueux cosmétiquement acceptable, au moins un tensio-actif anionique de type alkylgalactoside uronate et au moins un polymère amphotère.

Les alkylgalactoside uronates utilisables conformément à l'invention, répondent à la formule (I) suivante : 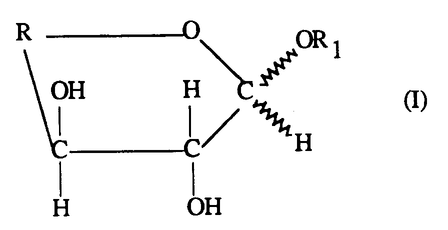 dans laquelle :
R₁ désigne un radical alkyle linéaire ou ramifié de 8 à 22 atomes de carbone.
R désigne un groupe
(i) 〉CH -CH(OH) - CO₂R₂ ou
(ii) 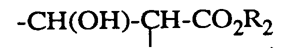 dont le carbone portant le groupe hydroxyle est relié à l'atome d'oxygène endocyclique; R₂ étant un hydrogène, un atome de métal alcalin, un métal alcalino-terreux ou un groupe ammonium quaternaire non substitué ou substitué par des radicaux alkyle, hydroxyalkyle ou dérivé d'aminoacides.

Le métal alcalin est notamment le sodium, le potassium et le métal alcalino-terreux est de préférence le magnésium. Comme sels d'ammonium quaternaires, on peut citer les sels d'ammoniaque, de triéthanolamine, de monoéthanolamine, de 2-amino 2-méthyl 1,3 propanediol, de 2-amino 2-méthyl 1-propanol; l'amino acide est notamment l'histidine, l'arginine ou la lysine.

On utilise de préférence les composés de formule (I) pour lesquels le radical R₁ désigne un alkyle en C₈-C₁₄ et plus particulièrement le radical décyle.

On utilise notamment les composés suivants :
Décyl α-D-galactopyranoside uronate de sodium : 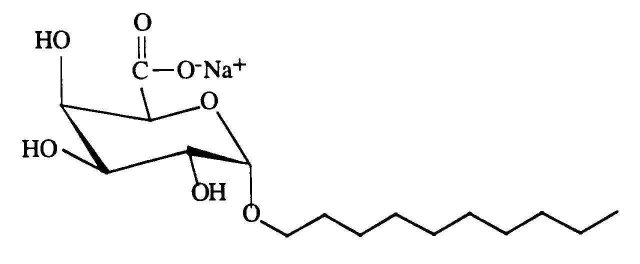
Décyl β-D-galactopyranoside uronate de sodium : 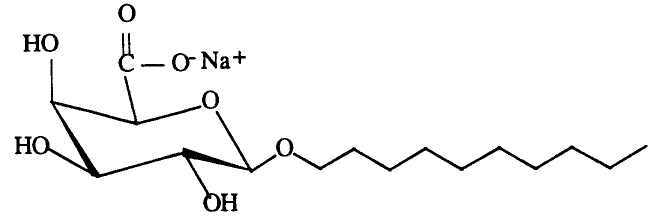
Décyl α-D-galactofuranoside uronate de sodium : 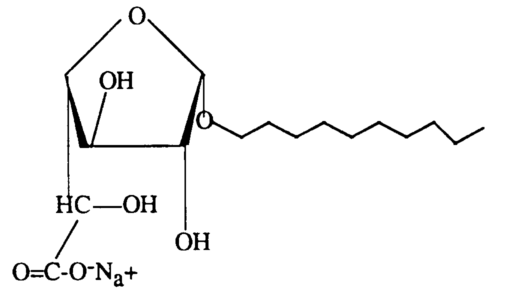
Décyl β-D-galactofuranoside uronate de sodim :

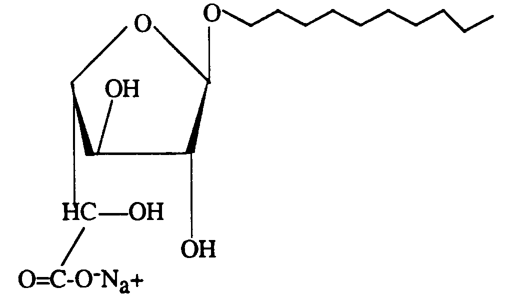

Les polymères amphotères utilisables conformément à l'invention sont choisis parmi les polymères comportant des motifs A et B répartis statistiquement dans la chaîne polymère où A désigne un motif dérivant d'un monomère comportant au moins un atome d'azote basique et B désigne un motif dérivant d'un monomère acide comportant un ou plusieurs groupements carboxyliques ou sulfoniques ou bien A et B peuvent désigner des groupements dérivant de monomères zwittérioniques de carboxybétaïnes ou de sulfobétaïnes;

A et B peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien A et B font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition indiquée ci-dessus plus particulièrement préférés sont choisis parmi les polymères suivants :
(1) les polymères résultants de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome d'azote basique tel que plus particulièrement les dialkylaminoalkylméthacrylate et acrylate, les dialkylaminoalkylméthacrylamide et acrylamide. De tels composés sont décrits dans le brevet américain n° 3 836 537.
(2) Les polymères dérivés de diallyldialkylammonium et d'au moins un monomère anionique tels que les polymères comportant environ 60 à environ 99% en poids d'unités dérivées d'un monomère de diallyldialkylammonium quaternaire dans lequel les groupements alkyle sont choisis indépendamment parmi les groupements alkyle ayant 1 à 18 atomes de carbone et dans lequel l'anion est dérivé d'un acide ayant une constante d'ionisation supérieure à 10⁻¹³ et 1 à 40% en poids de ce polymère, d'un monomère anionique choisi parmi les acides acrylique ou méthacrylique, le poids moléculaire de ce polymère étant compris entre environ 50 000 et 10 000 000 déterminé par chromatographie par perméation de gel. De tels polymères sont décrits dans la demande EP-A-269 243.
   Les polymères préférés sont entre autres les polymères comportant des groupements alkyle choisis parmi les groupements ayant 1 à 4 atomes de carbone et plus particulièrement des groupements méthyle, éthyle.
   Parmi ces polymères, les copolymères de chlorure de diméthyldiallylammonium ou de diéthyldiallylammonium et d'acide acrylique sont particulièrement préférés. Ces polymères sont par exemple vendus sous les dénominations "MERQUAT 280" et "MERQUAT 295" par la Société MERCK. On peut également utiliser les terpolymères de chlorure de diméthyldiallylammonium/acide acrylique/acrylamide vendu sous la dénomination "MERQUAT PLUS 3330" par la Société MERCK.
(3) Les polymères comportant des motifs dérivant
   a) d'au moins un monomère choisi parmi les acrylamides ou les méthacrylamides substitués à l'azote par un radical alkyle,
   b) d'au moins un comonomère acide contenant un ou plusieurs groupements carboxyliques réactifs, et
   c) au moins un comonomère basique tel que des esters à substituants amines primaire, secondaire, tertiaire et quaternaire des acides acrylique et méthacrylique et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertio-octyl acrylamide, le N-octylacrylamide, le N-décylacrylamide, le N-dodécylacrylamide ainsi que les méthacrylamides correspondants. Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone de l'acide maléïque ou de l'acide fumarique.
   Les comonomères basiques préférés sont des méthacrylates d'amino-éthyle, de butyl aminoéthyle, de N,N'-diméthylaminoéthyle, de N-tertio-butylaminoéthyle.
(4) les polyamino amides réticulés et alcoylés partiellement ou totalement dérivant de polyaminoamides de formule générale :

   ⁅OC―R₇―CO―Z⁆ (II)

   dans laquelle R₇ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalcoylène-polyamine bis-primaire, mono- ou bis-secondaire et de préférence représente :
   a) dans les proportions de 60 à 100 moles %, le radical

      ―NH⁅(CH₂)ₓ ― NH⁆ₙ (III)

      où x = 2 et n = 2 ou 3 ou bien x = 3 et n = 2
      ce radical dérivant de la diéthylène triamine, de la triéthylène tétramine ou de la dipropylène triamine;
   b) dans les proportions de 0 à 40 moles % le radical (III) ci-dessus, dans lequel x = 2 et n = 1 et qui dérive de l'éthylènediamine, ou le radical dérivant de la pipérazine : 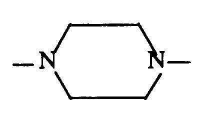
   c) dans les proportions de 0 à 20 moles % le radical
   - NH-(CH₂)₆ - NH - dérivant de l'hexaméthylènediamine, ces polyamino-amines étant réticulés par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4- et -2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(5) Les polymères comportant des motifs zwitterioniques de formule : 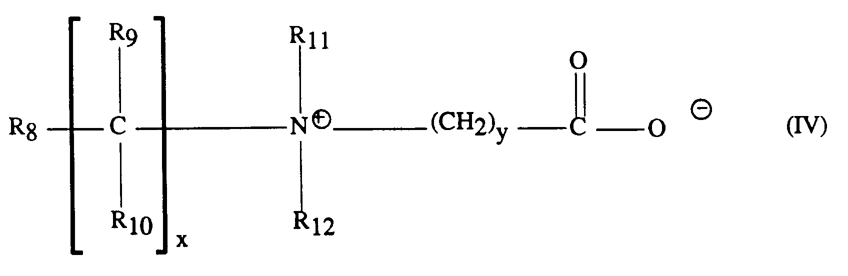 dans laquelle R₈ désigne un groupement insaturé polymérisable tel qu'un groupement acrylate, méthacrylate, acrylamide ou méthacrylamide, x et y représentent un nombre entier de 1 à 3, R₉ et R₁₀ représentent hydrogène, méthyle, éthyle ou propyle, R₁₁ et R₁₂ représentent un atome d'hydrogène ou un radical alkyle de telle façon que la somme des atomes de carbone dans R₁₁ et R₁₂ ne dépasse pas 10.
   Les polymères comprenant de telles unités peuvent également comporter des motifs dérivés de monomères non zwittérioniques tels que la vinylpyrrolidone, l'acrylate ou le méthacrylate de diméthyle ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de méthyle/diméthyl carboxyméthylammonio éthylméthacrylate de méthyle.
(6) Les polymères dérivés du chitosane comportant des motifs monomères répondant aux formules suivantes : 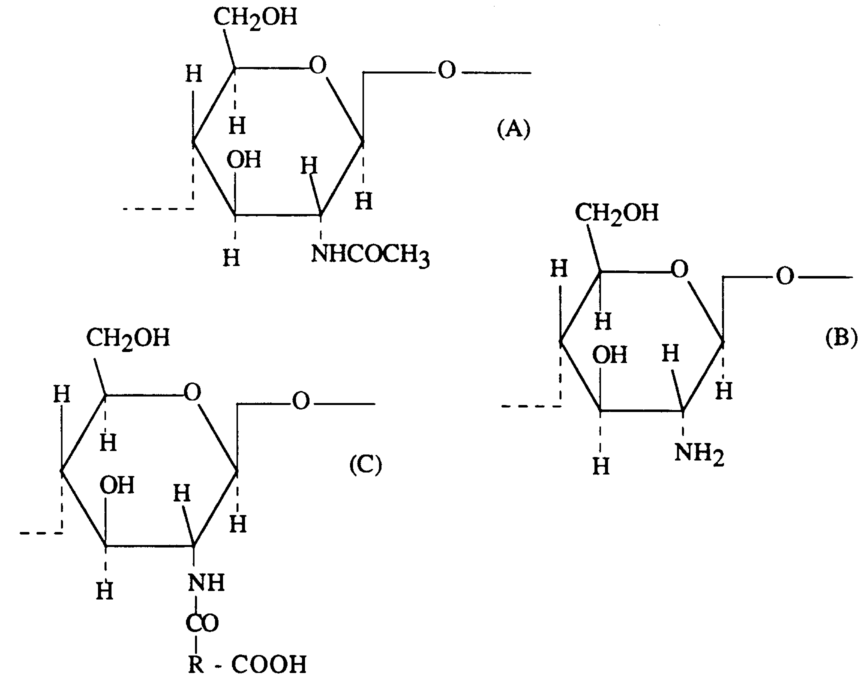 dans lesquelles le motif A est présent dans des proportions comprises entre 0 et 30%, B est présent dans des proportions comprises entre 5 et 50% et C est présent dans des proportions comprises entre 30 et 90%. Dans la formule C, R représente un radical de formule : 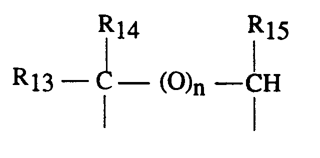 dans laquelle si n = O, R₁₃, R₁₄ et R₁₅, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₁₃, R₁₄ et R₁₅ étant dans ce cas un atome d'hydrogène; ou si n = 1, R₁₃, R₁₄ et R₁₅ représentent chacun un atome d'hydrogène, ainsi que les sels formés par ces composés avec des bases ou des acides.
(7) Les polymères dérivés de la N-carboxyalkylation du chitosane comme le N-carboxyméthyl chitosane ou le N-carboxybutyl chitosane vendu sous la dénomination "EVALSAN" par la Société JAN DEKKER.
(8) Les polymères répondant à la formule générales (V) sont décrits dans le brevet français 1 400 366 : 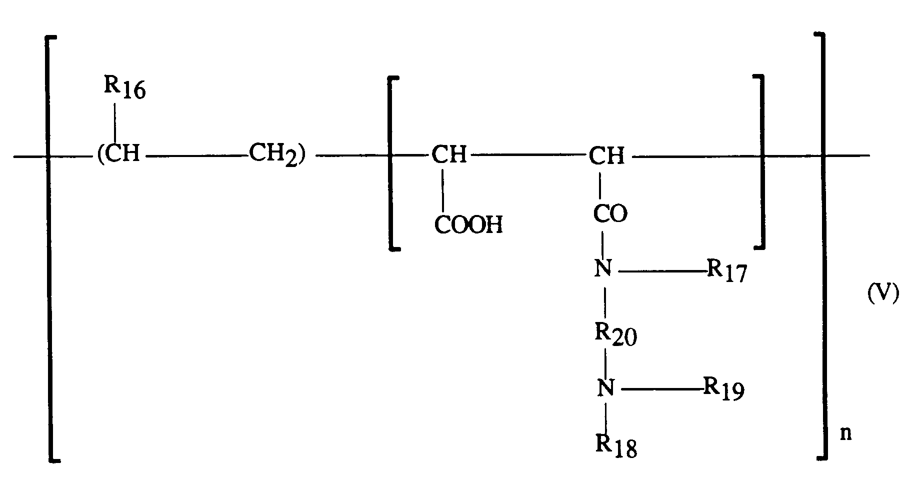 dans laquelle R₁₆ représente un atome d'hydrogène, un radical CH₃O, CH₃CH₂O, phényle, R₁₇ désigne de l'hydrogène ou un radical alkyle inférieur tel que méthyle, éthyle, R₁₈ désigne de l'hydrogène ou un radical alkyle inférieur tel que méthyle, éthyle, R₁₉ désigne un radical alkyle inférieur tel que méthyle, éthyle ou un radical répondant à la formule : -R₂₀-N (R₁₈)₂, R₂₀ représentant un groupement 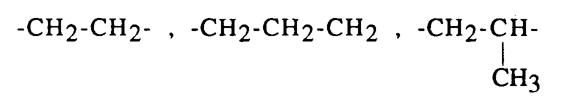 R₁₈ ayant les significations mentionnées ci-dessus. ainsi que les homologues supérieurs de ces radicaux et contenant jusqu'à 6 atomes de carbone.
(9) Des polymères amphotères du type -A-Z-A-Z choisis parmi
   a) les polymères obtenus par action de l'acide chloracétique ou le chloracétate de sodium sur les composés comportant au moins un motif de formule :

      -A-Z-A-Z-A- (VI)

      où A désigne un radical 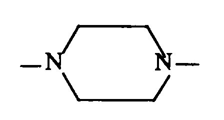 dans laquelle Z désigne le symbole B ou B', B ou B' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupement éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthane.
   b) Les polymères de formule -A-Z-A-Z- (VI') où A désigne un radical 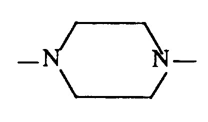 où Z désigne B ou B' et au moins une fois B'; B ayant la signification indiquée ci-dessus et B' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

Les alkylgalactoside uronates de formule (I) sont présents dans les compositions conformes à l'invention, dans des proportions comprises de préférence entre 1 et 50% en poids par rapport au poids total de la composition.

Les polymères amphotères définis précédemment sont présents dans des proportions comprises entre 0,01 et 10% en poids par rapport au poids total de la composition.

Si les compositions selon l'invention ne sont pas utilisés pour le lavage des matières kératiniques, la concentration totale en tensioactifs anioniques de formule (I) est comprise entre 1 et 10% et plus particulièrement entre 1 et 5% en poids par rapport au poids total de la composition. Ces compositions sont utilisées notamment comme compositions à rincer ou non, appliquées avant ou après un shampooing, une coloration, une décoloration, une permanente, un défrisage, dans une composition de coloration, de décoloration, de permanente ou de défrisage des cheveux.

Lorsque les compositions selon l'invention sont des compositions lavantes, elles contiennent les agents tensio-actifs de formule (I) en une concentration totale comprise entre 4 et 50% en poids et de préférence entre 8 et 40% en poids par rapport au poids total de la composition.

Les compositions peuvent contenir en plus de l'alkylgalactoside uronate, d'autres agents co-tensio-actifs de nature anionique, non-ionique, amphotère, zwitterionique ou cationique.

Parmi les agents tensio-actifs anioniques, on peut citer les sels alcalins, les sels d'ammonium, les sels d'amines, les sels d'aminoalcools, les sels de magnésium des composés suivants : les acides gras, les alkylsulfates, les alkyléthersulfates, les alkylamidoéthersulfates, les alkylarylpolyéthersulfates, les monoglycérides sulfates; les alkylsulfonates, les alkyléthersulfonates, les alkylamides sulfonates, les alkylarylsulfonates, les oléfines sulfonates, les paraffines sulfonates; les alkylsulfosuccinates, les alkyléthersulfosuccinates, les alkylamides sulfosuccinates; les alkylsulfosuccinamates; les alkylsulfoacétates; les alkylétherphosphates; les acylsarcosinates; les N-acyltaurates; les acylglutamates, les iséthionates.

Le radical alkyle ou acyle de ces différents composés est généralement constitué par une chaîne carbonée comportant de 10 à 20 atomes de carbone.

On peut également utiliser des agents tensio-actifs faiblement anioniques, tels que les acides alkylamides ou alkyléthers carboxyliques polyoxyalkylénés, tels que ceux comportant 2 à 50 groupements oxyde d'éthylène.

Les agents tensio-actifs non-ioniques sont plus particulièrement choisis parmi les alcools ou les α-diols ou les alkylphénols ou les acides gras polyéthoxylés ou polypropoxylés à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30.

On peut citer plus particulièrement les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amines grasses polyéthoxylées ayant de préférence 2 à 30 moles d'oxyde d'éthylène; les esters d'acides gras du sorbitan oxyéthylénés ayant de préférence 2 à 30 moles d'oxyde d'éthylène; les esters d'acides gras de sucre, les esters d'acides gras du polyéthylèneglycol, les esters d'acides gras de glycols, les oxydes d'amines tels que les oxydes d'alkyl(C₁₀-C₁₄) amines ou de N-acylamidopropylmorpholine.

Les agents tensio-actifs amphotères ou zwittérioniques préférés sont les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆)bétaïnes ou les alkyl(C₈-C₂₀)amidoalkyl(C₁-C₆)sulfobétaïnes.

On peut également citer les alkylpeptides, les alkylimidazolium bétaïnes.

Parmi les dérivés d'amines, on peut citer les produits commercialisés sous la dénomination "MIRANOL", tels que ceux décrits dans les brevets US-A-2.528.378 et 2.781.354 ou classés dans le dictionnaire CTFA, 3ème édition, 1982, sous les dénominations d'Amphocarboxyglycinates ou d'Amphocarboxypropionates.

Les agents tensio-actifs cationiques sont choisis parmi les sels d'ammonium quaternaires tels que les halogénures d'alkyl(C₈-C₂₂)triméthyl ammonium, les halogénures de dialkyl (C₈-C₂₂) diméthyl ammonium, les halogénures d'alkyl(C₈-C₂₂) diméthyl hydroxyéthyl ammonium.

Les co-tensio-actifs additionnels, peuvent représenter jusqu'à 50% du poids total des agents tensio-actifs présents dans la composition.

Le pH des compositions conformes à l'invention est généralement compris entre 2 et 10,5 plus particulièrement entre 3 et 8.

Dans la mesure où le milieu cosmétiquement acceptable de la composition selon l'invention est un milieu aqueux, il peut être constitué uniquement par de l'eau ou par un mélange d'eau et d'un solvant cosmétiquement acceptable, tel que des alcools inférieurs en C₁-C₄, comme l'éthanol, l'isopropanol, le n-butanol; les alkylèneglycols comme le propylèneglycol, les éthers de glycols.

Les compositions conformes à l'invention peuvent se présenter sous formes diverses telles que de liquide plus ou moins épaissi, de gel, de pain solide, d'émulsion (lait ou crème), de lotion hydroalcoolique, de dispersion et éventuellement être conditionnées en aérosol et être dispersées sous forme de mousse.

Les compositions sont par exemple des lotions, des laits ou des crèmes émollients, des lotions, des laits ou des crèmes pour les soins des matières kératiniques, des crèmes ou des laits démaquillants, des bases de fonds de teint, des lotions, des laits ou des crèmes antisolaires, des lotions, des laits ou des crèmes de bronzage artificiel, des crèmes ou des mousses de rasage, des lotions après rasage, des masques pour le visage, des produits de maquillage pour les yeux, des fards et fonds de teint pour le visage, des shampooings, des produits pour le bain ou la douche, des compositions à rincer ou non, à appliquer avant ou après un shampooing, une coloration, une décoloration, une permanente ou un défrisage, des compositions de coloration, de décoloration, de permanente ou de défrisage des cheveux.

Les compositions conformes à l'invention peuvent également contenir en plus divers additifs tels que des agents épaississants tels que des acides polyacryliques, des dérivés de cellulose, des esters d'acides gras et de polyéthylèneglycol, des séquestrants, des renforçateurs de mousse, des conservateurs, des parfums, des électrolytes, des corps gras tels que des alcools gras, des céramides, des huiles ou cires minérales, végétales, animales ou synthétiques, des filtres UV, des agents anti-radicaux libres, des agents nacrants, des biocides, des antibactériens, des agents antipelliculaires, des agents anti-séborrhéïques, des anti-parasitaires, des repellents, des colorants, des pigments, des oxydants, des réducteurs, des hydratants, des polymères, anioniques, non ioniques ou cationiques, des vitamines, des α-hydroxyacides.

Le traitement des matières kératiniques se fait par application sur ces matières d'une quantité cosmétiquement acceptable d'une composition telle que définie précédemment.

Le procédé de lavage et/ou de conditionnement des matières kératiniques et en particulier des cheveux conforme à l'invention consiste à appliquer sur ces matières au moins une composition telle que définie ci-dessus, cette application étant suivie éventuellement d'une étape de rinçage à l'eau.

Les compositions de lavage peuvent être utilisées comme shampooings mais également comme gels douche pour le lavage des cheveux et de la peau, auquel cas ils sont appliqués sur la peau et les cheveux humides qui sont rinçés après application.

Lorsque les compositions sont utilisées pour le conditionnement des cheveux, elles sont appliquées sur les cheveux humides, après quoi les cheveux sont soit séchés, soit après un temps de pose de 1 à 10 minutes, ils sont rincés l'eau. On constate que les cheveux humides se démêlent bien, que la mise en forme de la coiffure est facilitée et que celle-ci tient bien dans le temps.
Les exemples qui suivent sont destinés à illustrer l'invention sans pour autant présenter un caractère limitatif.

| **EXEMPLE 1** **SHAMPOOING** | |
|---|---|
| - Décyl - D-galactoside uronate de sodium | 25 g |
| - Copolymère N-octylacrylamide / méthylméthacrylate / hydroxypropyl méthacrylate / acide acrylique / Tert.butyl aminoéthyl méthacrylate, à 100 % MA vendu sous le nom de "AMPHOMER" par la société NATIONAL STARCH | 1 g |
| eau qsp | 100 g |
| pH ajusté à 7,5 par NaOH | |

| **EXEMPLE 2** **SHAMPOOING** | |
|---|---|
| - Décyl - D-galactoside uronate de sodium | 15 g |
| - cocoyl-bétaine en sol à 32 % | 5 g MA |
| - Diuréthane d'alcools (C₁₆/C₁₈)oxyéthyléné et oxypropyléné à 100 % MA "DAPRAL T 212" (AKZO) | 3 g |
| - copolymère méthacrylate d'alkyl (C₁-C₁₈) méthacryloyl bétaine - sol à 50 % (EtOH) "AMERSETTE" (Mitsubishi) | 1 g |
| eau qsp | 100 g |
| pH ajusté à 6,5 par HCl | |

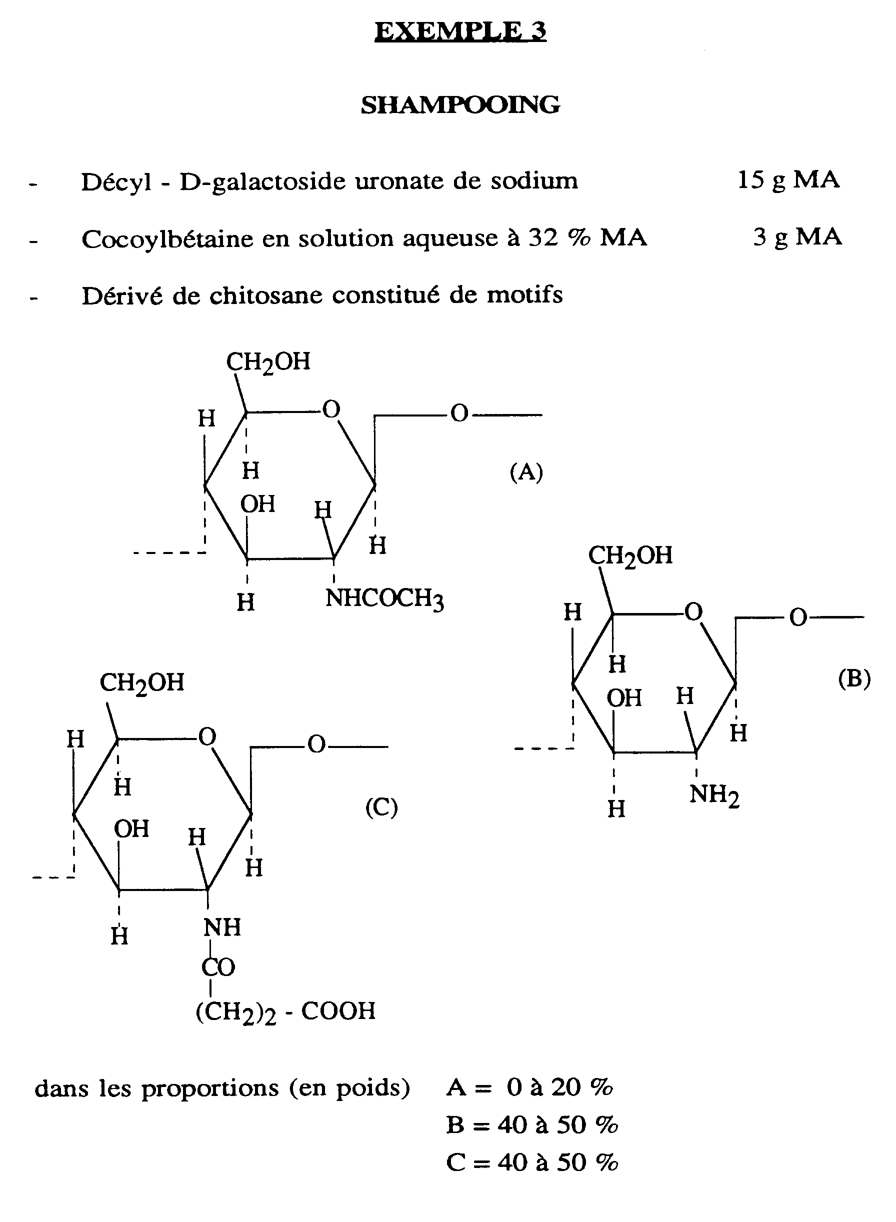

| **EXEMPLE 4** **APRES SHAMPOOING** | |
|---|---|
| - Décyl - D-galactoside uronate de sodium | 2 g MA |
| - Copolymère chlorure de diméthyl diallyl ammonium / acide acrylique (80/20) en solution aqeuse à 35 % vendu sous le nom de "MERQUAT 280" par la Société MERCK | 5 g MA |
| Colorant, parfum, conservateur | |
| eau qsp | 100 g |
| pH ajusté à 7 par NaOH | |

## Revendications

1. Composition cosmétique, caractérisée par le fait qu'elle contient dans un milieu aqueux cosmétiquement acceptable :
(A) au moins un agent tensio-actif anionique du type alkylgalactoside uronate de formule : 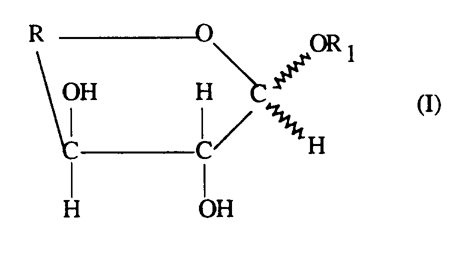 R₁ désigne un radical alkyle linéaire ou ramifié de 8 à 22 atomes de carbone.
R désigne un groupe
(i) 〉CH -CH(OH) - CO₂R₂ ou
(ii) 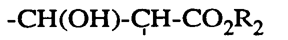 dont le carbone portant le groupe hydroxyle est relié à l'atome d'oxygène endocyclique; R₂ étant un hydrogène, un métal alcalin, un métal alcalino-terreux ou un groupe ammonium quaternaire non substitué ou substitué par des alkyles ou des hydroxyalkyles ou un groupement ammonium quaternaire dérivé d'amino-acides.
**(B)** au moins un polymère amphotère.

2. Composition selon la revendication 1, carctérisée par le fait que dans la formule (I), le radical R₂ désigne le sodium ou le potassium; le magnésium; un groupe ammonium quaternaire dérivé d'ammoniaque de triéthanolamine, de monoéthanolamine, de 2-amino 2-méthyl 1,3-propanediol, de 2-méthyl 2-amino 1-propanol, d'histidine, d'arginine ou de lysine.

3. Composition selon la revendication 1 ou 2, caractérisée par le fait que le composé de formule (I) est choisi parmi ceux pour lesquels R₁ désigne un alkyle en C₈-C₁₄.

4. Composition selon l'une quelconque des revendications 1 à 3, caractérisée par le fait que les composés de formule (I) sont choisis parmi ceux pour lesquels R₁ désigne le radical décyle.

5. Composition selon la revendication 4, caractérisée par le fait que le composé de formule (I) est choisi parmi :
le décyl α-D-galactopyranoside uronate de sodium
le décyl β-D-galactopyranoside uronate de sodium
le décyl α-D-galactofuranoside uronate de sodium
le décyl β-D-galactofuranoside uronate de sodium.

6. Composition selon l'une quelconque des revendications 1 à 4, caractérisée en ce que le polymère amphotère est choisi parmi les polymères comportant des motifs A et B répartis statistiquement dans la chaîne polymère où
A désigne un motif dérivant d'un monomère comportant au moins un atome d'azote basique et B désigne un motif dérivant d'un monomère acide comportant un ou plusieurs groupements carboxyliques ou sulfoniques;
ou bien A et B peuvent désigner des groupements dérivant de monomères zwittérioniques de carboxybétaïne ou de sulfobétaïne;
A et B peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné;
ou bien A et B font partie d'une chaîne d'un polymère à motif éthylène α,β-dicarboxylique dont l'un des groupements carboxylique a été amené à réagir avec une polyamine comportant un ou plusieurs groupements amine primaire ou secondaire.

7. Composition selon la revendication 6, caractérisée en ce que les polymères amphotères sont choisis parmi les polymères suivants :
(1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alphachloracrylique, et d'un monomère basique dérivé d'un composé vinylique substitué contenant au moins un atome d'azote basique tel que plus particulièrement les dialkylaminoalkylméthacrylate et acrylate, les dialkylaminoalkylméthacrylamide et acrylamide;
(2) les polymères dérivés de diallyldialkylammonium et d'au moins un monomère anionique tels que les polymères comportant environ 60 à environ 99% en poids d'unités dérivées d'un monomère de diallyldialkylammonium quaternaire dans lequel les groupements alkyle sont choisis indépendamment parmi les groupements alkyle ayant 1 à 18 atomes de carbone et dans lequel l'anion est dérivé d'un acide ayant une constante d'ionisation supérieure à 10⁻¹³ et 1 à 40% en poids de ce polymère, d'un monomère anionique choisi parmi les acides acrylique ou méthacrylique, le poids moléculaire de ce polymère étant compris entre environ 50 000 et 10 000 000 déterminé par chromatographie par perméation de gel;
(3) les polymères comportant des motifs dérivant
a) d'au moins un monomère choisi parmi les acrylamides ou les méthacrylamides substitués à l'azote par un radical alkyle,
b) d'au moins un comonomère acide contenant un ou plusieurs groupements carboxyliques réactifs, et
c) au moins un comonomère basique tel que des esters à substituants amines primaire, secondaire, tertiaire et quaternaire des acides acrylique et méthacrylique et le produit de quaternisation du méthacrylate de diméthylaminoéthyle avec le sulfate de diméthyle ou diéthyle.
(4) les polyamino amides réticulés et alcoylés partiellement ou totalement dérivant de polyaminoamides de formule générale :
⁅OC―R₇―CO―Z⁆ (II)
dans laquelle R₇ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalcoylène-polyamine bis-primaire, mono- ou bis-secondaire et de préférence représente :
a) dans les proportions de 60 à 100 moles %, le radical
― NH⁅(CH₂)ₓ ― NH⁆ₙ (III)
où x = 2 et n = 2 ou 3 ou bien x = 3 et n = 2
ce radical dérivant de la diéthylène triamine, de la triéthylène tétramine ou de la dipropylène triamine;
b) dans les proportions de 0 à 40 moles % le radical (III) ci-dessus, dans lequel x = 2 et n = 1 et qui dérive de l'éthylènediamine, ou le radical dérivant de la pipérazine : 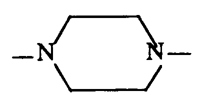
c) dans les proportions de 0 à 20 moles % le radical - NH (CH₂)-₆ - NH - dérivant de l'hexaméthylènediamine, ces polyamino-amines étant réticulés par addition d'un agent réticulant bifonctionnel choisi parmi les épihalohydrines, les diépoxydes, les dianhydrides, les dérivés bis insaturés au moyen de 0,025 à 0,35 mole d'agent réticulant par groupement amine du polyaminoamide et alcoylés par action d'acide acrylique, d'acide chloracétique ou d'une alcane sultone ou de leurs sels;
(5) les polymères comportant des motifs zwitterioniques de formule : 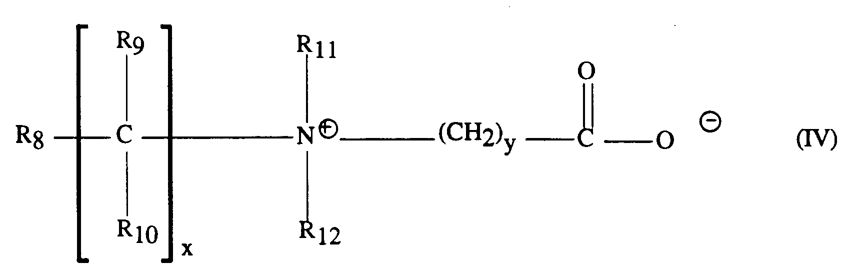 dans laquelle R₈ désigne un groupement insaturé polymérisable tel qu'un groupement acrylate, méthacrylate, acrylamide ou méthacrylamide, x et y représentent un nombre entier de 1 à 3, R₉ et R₁₀ représentant hydrogène, méthyle, éthyle ou propyle, R₁₁ et R₁₂ représentant un atome d'hydrogène ou un radical alkyle de telle façon que la somme des atomes de carbone dans R₁₁ et R₁₂ ne dépasse pas 10;
les polymères comprenant de telles unités peuvent également comporter des motifs dérivés de monomères non zwittérioniques tels que la vinylpyrrolidone, l'acrylate ou le méthacrylate de diméthyl ou diéthylaminoéthyle ou des alkyl acrylates ou méthacrylates, des acrylamides ou méthacrylamides ou l'acétate de vinyle;
(6) les polymères dérivés du chitosane comportant des motifs monomères répondant aux formules suivantes : 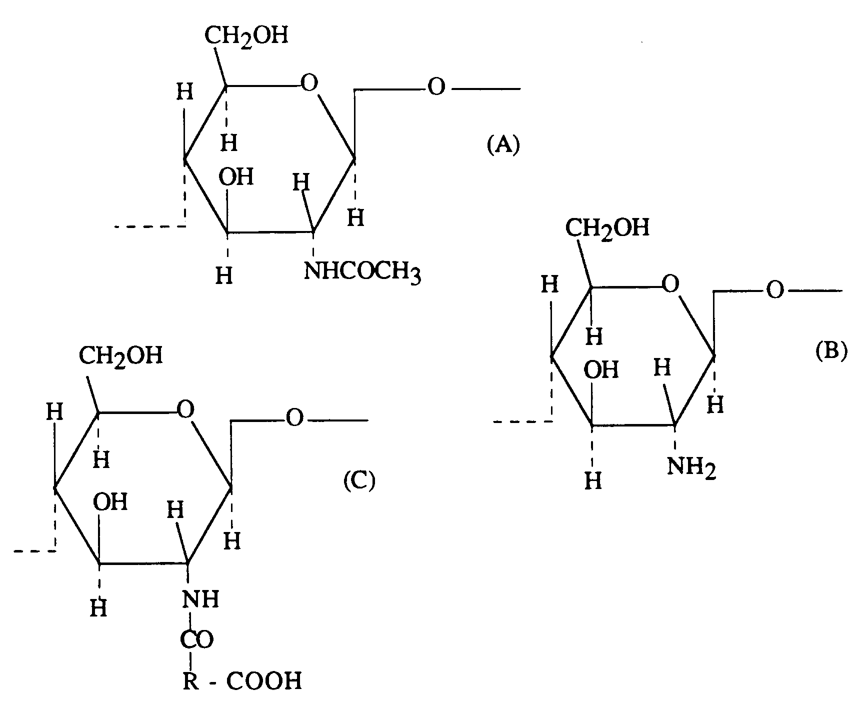 dans lesquelles le motif A est présent dans des proportions comprises entre 0 et 30%, B est présent dans des proportions comprises entre 5 et 50% et C est présent dans des proportions comprises entre 30 et 90%; dans la formule C, R représente un radical de formule : 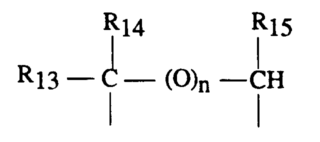 dans laquelle si n = O, R₁₃, R₁₄ et R₁₅ identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₁₃, R₁₄ et R₁₅ étant dans ce cas un atome d'hydrogène; ou si n = 1, R₁₃, R₁₄ et R₁₅ représentent chacun un atome d'hydrogène, ainsi que les sels formés par ces composés avec des bases ou des acides;
(7) les polymères dérivés de la N-carboxyalkylation du chitosane
(8) les polymères répondant à la formule générale (V) : 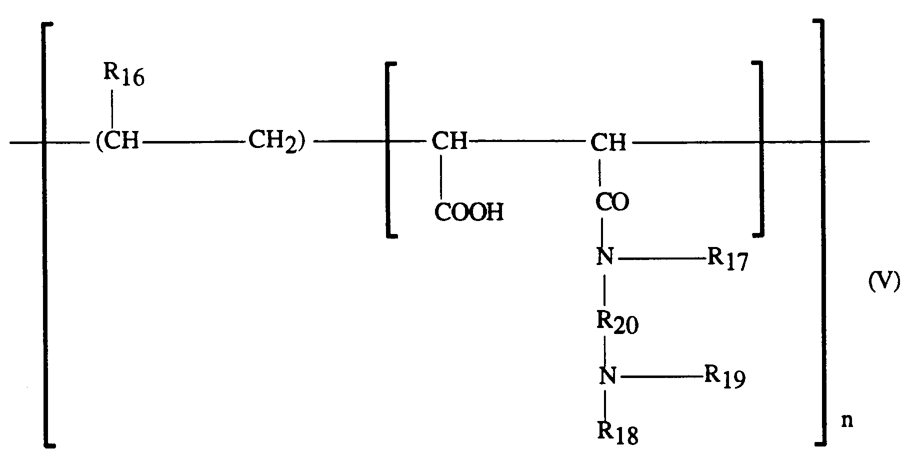 dans laquelle R₁₆ représente un atome d'hydrogène, un radical CH₃O, CH₃CH₂O, phényle, R₁₇ désigne de l'hydrogène ou un radical alkyle inférieur tel que méthyle, éthyle, R₁₈ désigne de l'hydrogène ou un radical alkyle inférieur tel que méthyle, éthyle, R₁₉ désigne un radical alkyle inférieur tel que méthyle, éthyle ou un radical répondant à la formule : -R₂₀-N (R₁₈)₂, R₂₀ représentant un groupement 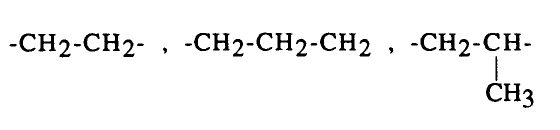 R₁₈ ayant les significations indiuqées ci-dessus,
ainsi que les homologues supérieurs de ces radicaux et contenant jusqu'à 6 atomes de carbone;
(9) les polymères amphotères du type -A-Z-A-Z choisis parmi
a) les polymères obtenus par action de l'acide chloracétique ou le chloracétate de sodium sur les composés comportant au moins un motif de formule :
-A-Z-A-Z-A- (VI)
où A désigne un radical 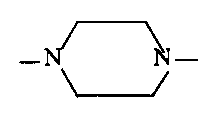 dans laquelle Z désigne le symbole B ou B', B ou B' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupement éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthane;
b) les polymères de formule -A-Z-A-Z- (VI') où A désigne un radical 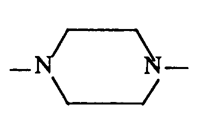 où Z désigne B ou B' et au moins une fois B'; B ayant la signification indiquée ci-dessus et B' est un radical bivalent qui est un radical alkylène à chaîne droite ou ramifiée ayant jusqu'à 7 atomes de carbone dans la chaîne principale, substituée ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction de l'acide chloracétique ou du chloracétate de soude.

8. Composition selon l'une quelconque des revendications 1 à 7, caractérisée par le fait que les agents tensio-actifs anioniques de formule (I) sont présents dans des proportions comprises entre 1 et 50% en poids et les polymères amphotères sont présents dans des proportions comprises entre 0,01 et 10%, les pourcentages en poids étant exprimés par rapport au poids total de la composition.

9. Composition de conditionnement des matières kératiniques selon l'une quelconque des revendications 1 à 8, caractérisée par le fait que la concentration en agents tensio-actifs anioniques de formule (I) est comprise entre 1 et 10% en poids par rapport au poids total de la composition.

10. Composition de lavage des matières kératiniques selon l'une quelconque des revendications 1 à 8, caractérisée par le fait que la concentration en tensio-actifs anioniques de formule (I) est comprise entre 4 et 50% en poids par rapport au poids total de la composition.

11. Composition selon l'une quelconque des revendications 1 à 10, caractérisée par le fait qu'elle contient en plus un co-tensio-actif additionnel du type anionique, non-ionique, amphotère, zwitterionique ou cationique dans une proportion allant jusqu'à 50% du poids total des agents tensio-actifs présents dans la composition.

12. Composition selon la revendication 11, caractérisée par le fait que le co-tensio-actif anionique additionnel est choisi parmi les sels alcalins, les sels d'ammonium, les sels d'amines, les sels d'aminoalcools, les sels de magnésium des composés suivants : les acides gras, les alkylsulfates, les alkyléthersulfates, les alkylamidoéthersulfates, les alkylarylpolyéthersulfates, les monoglycérides sulfates; les alkylsulfonates, les alkyléthersulfonates, les alkylamides sulfonates, les alkylarylsulfonates, les oléfines sulfonates, les paraffines sulfonates; les alkylsulfosuccinates, les alkyléthersulfosuccinates, les alkylamides sulfosuccinates; les alkylsulfosuccinamates; les alkylsulfoacétates; les alkylétherphosphates; les acylsarcosinates; les acylglutamates; les N-acyltaurates; les iséthionates; le radical alkyle ou acyle étant constitué d'une chaîne carbonée comportant de 10 à 20 atomes de carbone ou bien des acides alkylamides ou alkyléthers carboxyliques polyoxyalkylénés.

13. Composition selon la revendication 11, caractérisée par le fait que le co-tensio-actif non-ionique additionnel est choisi parmi les alcools, les α-diols ou les alkylphénols et les acides gras polyéthoxylés, ou polypropoxylés à chaîne grasse comportant 8 à 18 atomes de carbone, le nombre de groupements oxyde d'éthylène ou oxyde de propylène étant compris entre 2 et 50 et le nombre de groupements glycérol étant compris entre 2 et 30, les copolymères d'oxydes d'éthylène et du propylène, les condensats d'oxydes d'éthylène et de propylène sur des alcools gras, les amides gras polyéthoxylés, les amines grasses polyéthoxylées, les esters d'acide gras du sorbitan oxyéthylénés, les esters d'acides gras de sucre, les esters d'acides gras de glycols, les oxydes d'amines.

14. Composition selon la revendicaton 11, caractérisée par le fait que le co-tensio-actif amphotère additionnel est choisi parmi les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆)bétaïnes ou les alkyl(C₈-C₂₀)amidoalkyl(C₁-C₆)sulfobétaïnes; les alkylpeptides; les alkylimidazolium bétaïnes.

15. Composition selon la revendication 11, caractérisée par le fait que le co-tensio-actif cationique est choisi parmi les sels d'ammonium quaternaire.

16. Composition selon l'une quelconque des revendications 1 à 15, caractérisée par le fait que le milieu cosmétiquement acceptable est constitué par de l'eau ou un mélange d'eau et d'un solvant cosmétiquement acceptable.

17. Composition selon l'une quelconque des revendications 1 à 16, caractérisée par le fait qu'elle se présente sous forme de liquide plus ou moins épaissi, de gel, d'émulsion, de lotion hydroalcoolique, de dispersion ou de mousse aérosol.

18. Composition selon l'une quelconque des revendications 1 à 17, caractérisée par le fait qu'elle contient en plus des additifs choisis parmi les renforçateurs de mousse, les épaississants, les séquestrants, les électrolytes, les parfums, les conservateurs, les alcools gras, les huiles ou cires minérales, végétales ou animales ou synthétiques, les céramides, des filtres UV, des agents anti-radicaux libres, des agents nacrants, des biocides, des antibactériens, des agents antipelliculaires, des agents anti-séborrhéïques, des anti-parasitaires, des repellents, des colorants, des pigments, des oxydants, des réducteurs, des hydratants, des polymères, anioniques, non ioniques ou cationiques, des vitamines, des α-hydroxyacides.

19. Utilisation de la composition telle que définie dans l'une quelconque des revendications 1 à 18 pour le traitement et/ou le lavage des matières kératiniques constituées par les cheveux ou la peau.

20. Procédé de lavage et/ou de conditionnement cosmétique des cheveux ou de la peau, caractérisé par le fait qu'il consiste à appliquer sur la peau ou les cheveux une quantité efficace de composition selon l'une quelconque des revendications 1 à 18, cette application étant éventuellement suivie d'un rinçage à l'eau.

## Claims

1. Cosmetic composition, characterized in that it contains, in a cosmetically acceptable aqueous medium:
(A) at least one anionic surfactant of alkylgalactoside uronate type of formula: 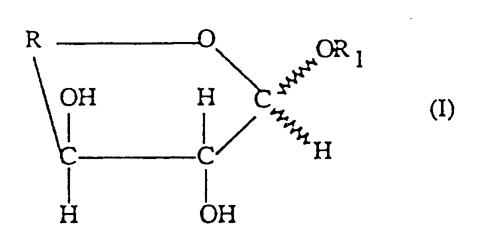 [lacuna]
R₁ denotes a linear or branched alkyl radical containing 8 to 22 carbon atoms,
R denotes a group
(i) 〉CH -CH(OH) - CO₂R₂ or
(ii) 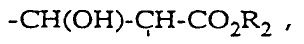 in which the carbon carrying the hydroxyl group is connected to the endocyclic oxygen atom; R₂ being a hydrogen, an alkali metal, an alkaline-earth metal or a quaternary ammonium group which is unsubstituted or substituted by alkyls or hydroxyalkyls or a quaternary ammonium group derived from amino acids,
(B) at least one amphoteric polymer.

2. Composition according to Claim 1, characterized in that, in the formula (I), the radical R₂ denotes sodium or potassium; magnesium; or a quaternary ammonium group derived from ammonia [lacuna] triethanolamine, monoethanolamine, 2-amino-2-methyl-1,3-propanediol, 2-methyl-2-amino-1-propanol, histidine, arginine or lysine.

3. Composition according to Claim 1 or 2, characterized in that the compound of formula (I) is chosen from those in which R₁ denotes a C₈-C₁₄ alkyl.

4. Composition according to any one of Claims 1 to 3, characterized in that the compounds of formula (I) are chosen from those in which R₁ denotes the decyl radical.

5. Composition according to Claim 4, characterized in that the compound of formula (I) is chosen from:
sodium decyl α-D-galactopyranoside uronate
sodium decyl β-D-galactopyranoside uronate
sodium decyl α-D-galactofuranoside uronate
sodium decyl β-D-galactofuranoside uronate.

6. Composition according to any one of Claims 1 to 4, characterized in that the amphoteric polymer is chosen from
polymers containing units A and B distributed statistically in the polymer chain, where
A denotes a unit deriving from a monomer containing at least one basic nitrogen atom and B denotes a unit deriving from an acidic monomer containing one or a number of carboxyl or sulfonic groups;
or else A and B can denote groups deriving from carboxybetaine or sulfobetaine zwitterionic monomers;
A and B can also denote a cationic polymer chain containing primary, secondary, tertiary or quaternary amine groups, in which chain at least one of the amine groups carries a carboxyl or sulfonic group connected via a hydrocarbon radical;
or else A and B form part of a chain of a polymer containing an ethylene-α,β-dicarboxyl unit in which one of the carboxyl groups has been brought to react with a polyamine containing one or a number of primary or secondary amine groups.

7. Composition according to Claim 6, characterized in that the amphoteric polymers are chosen from the following polymers:
(1) polymers resulting from the copolymerization of a monomer derived from a vinyl compound carrying a carboxyl group, such as more particularly acrylic acid, methacrylic acid, maleic acid and alpha-chloroacrylic acid, and of a basic monomer derived from a substituted vinyl compound containing at least one basic nitrogen atom, such as more particularly dialkylaminoalkyl methacrylate and acrylate and dialkylaminoalkylmethacrylamide and -acrylamide;
(2) polymers derived from diallyldialkylammonium and from at least one anionic monomer, such as polymers containing approximately 60 to approximately 99% by weight of units derived from a quaternary diallyldialkylammonium monomer in which the alkyl groups are chosen independently from alkyl groups having 1 to 18 carbon atoms and in which the anion is derived from an acid having an ionization constant of greater than 10⁻¹³ and 1 to 40% by weight of this polymer of an anionic monomer chosen from acrylic or methacrylic acids [sic], the molecular weight of this polymer being between approximately 50,000 and 10,000,000, determined by gel permeation chromatography;
(3) polymers containing units deriving
a) from at least one monomer chosen from acrylamides or methacrylamides substituted on the nitrogen with an alkyl radical,
b) from at least one acidic comonomer containing one or a number of reactive carboxyl groups, and
c) [lacuna] at least one basic comonomer, such as esters containing primary, secondary, tertiary and quaternary amine substituents of acrylic and methacrylic acids and the product of quaternization of dimethylaminoethyl methacrylate with dimethyl or diethyl sulfate.
(4) polyaminoamides, partially or completely crosslinked and alkylated, deriving from polyaminoamides of general formula:
⁅OC―R₇―CO―Z⁆ (II)
in which R₇ represents a divalent radical derived from a saturated dicarboxylic acid, from a mono- or dicarboxylic aliphatic acid containing an ethylenic double bond, from an ester of these acids with a lower alkanol having 1 to 6 carbon atoms or from a radical deriving from the addition of any one of the said acids with [sic] a bis-primary or bis-secondary amine, and Z denotes a radical of a polyalkylenepoly(bis-primary or mono- or bis-secondary amine) and preferably represents:
a) in proportions of 60 to 100 mol %, the radical
― NH⁅(CH₂)ₓ ― NH⁆ₙ (III)
where x = 2 and n = 2 or 3 or else x = 3 and n = 2 this radical deriving from diethylenetriamine, triethylenetetramine or dipropylenetriamine;
b) in proportions of 0 to 40 mol %, the above radical (III) in which x = 2 and n = 1 and which derives from ethylenediamine, or the radical deriving from piperazine: 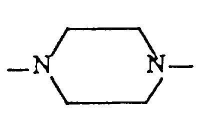
c) in proportions of 0 to 20 mol %, the radical -NH(CH₂)-₆-NH-[sic] deriving from hexamethylenediamine, these polyaminoamines [sic] being crosslinked by addition of a bifunctional crosslinking agent chosen from epihalohydrines, diepoxides, dianhydrides and bis-unsaturated derivatives, by means of 0.025 to 0.35 mol of cross-linking agent per amine group of the polyaminoamide and alkylated by the action of acrylic acid, chloroacetic acid or an alkanesultone or their salts;
(5) polymers containing zwitterionic units of formula: 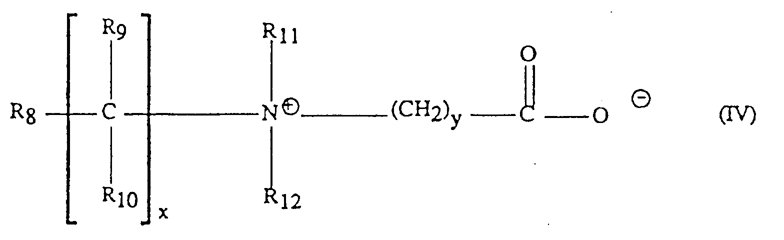 in which R₈ denotes a polymerizable unsaturated group such as an acrylate, methacrylate, acrylamide or methacrylamide group, x and y represent an integer from 1 to 3, R₉ and R₁₀ representing [sic] hydrogen, methyl, ethyl or propyl, and R₁₁ and R₁₂ representing [sic] a hydrogen atom or an alkyl radical such that the sum of the carbon atoms in R₁₁ and R₁₂ does not exceed 10;
the polymers comprising such units can also contain units derived from nonzwitterionic monomers such as vinylpyrrolidone or dimethyl- or diethylaminoethyl acrylate or methacrylate or alkyl acrylates or methacrylates, acrylamides or methacrylamides or vinyl acetate;
(6) polymers derived from chitosan containing monomer units corresponding to the following formulae: 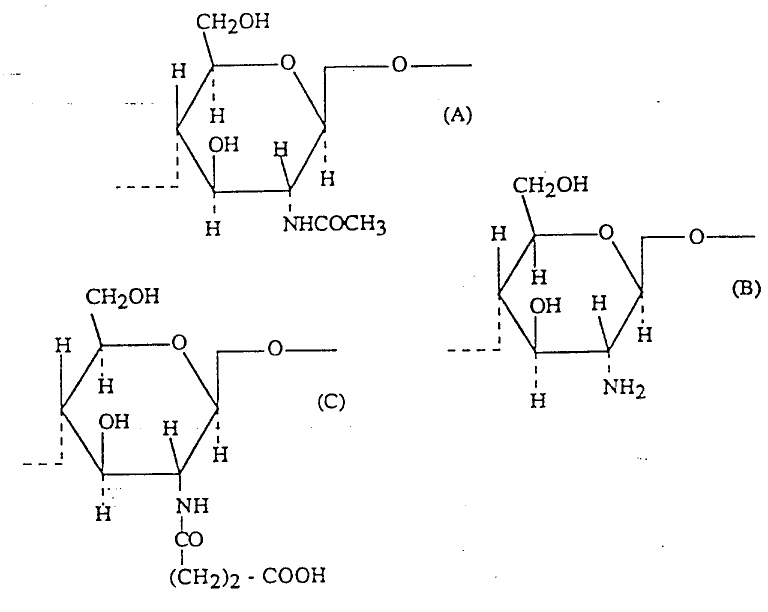 in which the A unit is present in proportions of between 0 and 30%, B is present in proportions of between 5 and 50 % and C is present in proportions of between 30 and 90%; in the formula C, R represents a radical of formula: 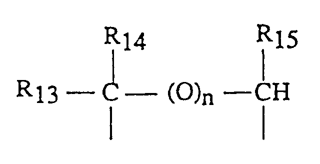 in which, if n = O [sic], R₁₃, R₁₄ and R₁₅, which are identical or different, each represent a hydrogen atom, a methyl, hydroxyl, acetoxy or amino residue, a monoalkylamine residue or a dialkylamine residue, optionally interrupted by one or a number of nitrogen atoms and/or optionally substituted with one or a number of amine, hydroxyl, carboxyl, alkylthio or sulfonic groups, or an alkylthio residue in which the alkyl group carries an amino residue, at least one of the radicals R₁₃, R₁₄ and R₁₅ in this case being a hydrogen atom; or, if n = 1, R₁₃, R₁₄ and R₁₅ each represent a hydrogen atom, as well as the salts formed by these compounds with bases or acids;
(7) polymers derived from the N-carboxyalkylation of chitosan;
(8) polymers corresponding to the general formula (V): 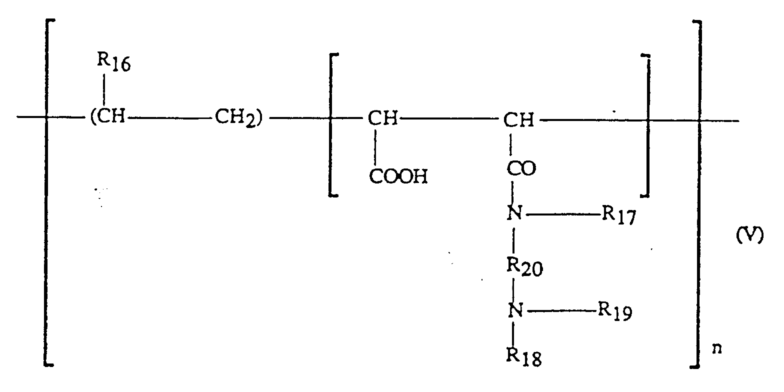 in which R₁₆ represents a hydrogen atom or a CH₃O, CH₃CH₂O or phenyl radical, R₁₇ denotes hydrogen or a lower alkyl radical such as methyl or ethyl, R₁₈ denotes hydrogen or a lower alkyl radical such as methyl or ethyl, and R₁₉ denotes a lower alkyl radical such as methyl or ethyl or a radical corresponding to the formula: -R₂₀-N(R₁₈)₂, R₂₀ representing a group 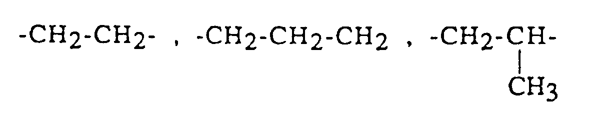 R₁₈ having the meanings mentioned above,
as well as the higher homologs of these radicals containing up to 6 carbon atoms;
(9) amphoteric polymers of the -A-Z-A-Z [sic] type chosen from
a) polymers obtained by the action of chloroacetic acid or sodium chloroacetate on compounds containing at least one unit of the formula:
-A-Z-A-Z-A- (VI)
where A denotes a radical 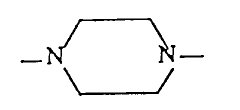 in which Z denotes the symbol B or B', B or B', which are identical or different, denote a divalent radical which is a straight- or branched-chain alkylene radical containing up to 7 carbon atoms in the main chain, which chain is unsubstituted or substituted with hydroxyl groups, and which can contain, in addition, oxygen, nitrogen and sulfur atoms and 1 to 3 aromatic and/or heterocyclic rings; the oxygen, nitrogen and sulfur atoms being present in the form of an ether, thioether, sulfoxide, sulfone, sulfonium, alkylamine or alkenylamine group or of hydroxyl, benzylamine, amine oxide, quaternary ammonium, amide, imide, alcohol, ester and/or urethane groups;
b) polymers of formula -A-Z-A-Z- (VI') where A denotes a radical 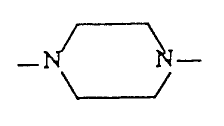 where Z denotes B or B', and B' at least once; B having the meaning stated above and B' is a divalent radical which is a straight- or branched-chain alkylene radical having up to 7 carbon atoms in the main chain, which chain is unsubstituted or substituted with one or a number of hydroxyl radicals and which contains one or a number of nitrogen atoms, the nitrogen atom being substituted with an alkyl chain optionally interrupted by an oxygen atom and necessarily containing one or a number of carboxyl functional groups and one or a number of hydroxyl functional groups, and converted to a betaine by reaction with chloroacetic acid or sodium chloroacetate.

8. Composition according to any one of Claims 1 to 7, characterized in that the anionic surfactants of formula (I) are present in proportions of between 1 and 50% by weight and the amphoteric polymers are present in proportions of between 0.01 and 10%, the percentages by weight being expressed with respect to the total weight of the composition.

9. Composition for conditioning keratinous substances according to any one of Claims 1 to 8, characterized in that the concentration of anionic surfactants of formula (I) is between 1 and 10% by weight with respect to the total weight of the composition.

10. Composition for washing keratinous substances according to any one of Claims 1 to 8, characterized in that the concentration of anionic surfactants of formula (I) is between 4 and 50% by weight with respect to the total weight of the composition.

11. Composition according to any one of Claims 1 to 10, characterized in that it furthermore contains an additional cosurfactant of anionic, nonionic, amphoteric, zwitterionic or cationic type in a proportion ranging up to 50% of the total weight of the surfactants present in the composition.

12. Composition according to Claim 11, characterized in that the additional anionic cosurfactant is chosen from the alkali metal salts, the ammonium salts, the amine salts, the aminoalcohol salts or the magnesium salts of the following compounds: the fatty acids, alkyl sulfates, alkyl ether sulfates, alkylamidoether sulfates, alkylarylpolyether sulfates or monoglyceride sulfates; the alkylsulfonates, alkylethersulfonates, alkylamidesulfonates, alkylarylsulfonates, olefinsulfonates or paraffinsulfonates; the alkylsulfosuccinates, the alkylethersulfosuccinates or the alkylamidesulfosuccinates; the alkylsulfosuccinamates; the alkylsulfoacetates; the alkyl ether phosphates; the acylsarcosinates; the N-acyltaurates; or the isethionates; the alkyl or acyl radical consisting of a carbon chain containing from 10 to 20 carbon atoms or else polyoxyalkylenated alkyl amide or alkyl ether carboxylic acids.

13. Composition according to Claim 11, characterized in that the additional nonionic cosurfactant is chosen from the alcohols, the α-diols or the alkylphenols and the polyethoxylated or polypropoxylated fatty acids, with a fatty chain containing 8 to 18 carbon atoms, the number of ethylene oxide or propylene oxide groups being between 2 and 50 and the number of glycerol groups being between 2 and 30 [sic], the copolymers of ethylene oxide and of the [sic] propylene oxide, the condensates of ethylene oxide and of propylene oxide with fatty alcohols, the polyethoxylated fatty amides, the polyethoxylated fatty amines, the oxyethylenated fatty acid esters of sorbitan, the fatty acid esters of sugar [sic], the fatty acid esters of glycols, or the amine oxides.

14. Composition according to Claim 11, characterized in that the additional amphoteric cosurfactant is chosen from the derivatives of secondary or tertiary aliphatic amines, in which the aliphatic radical is a linear or branched chain containing 8 to 18 carbon atoms and which contains at least one water-solubilizing carboxylate, sulfonate, sulfate, phosphate or phosphonate anionic group; the (C₈-C₂₀)alkylbetaines, the sulfobetaines, the (C₈-C₂₀)alkylamido(C₁-C₆)alkylbetaines or the (C₈-C₂₀)-alkylamido(C₁-C₆)alkylsulfobetaines; the alkylpeptides; or the alkylimidazolium betaines.

15. Composition according to Claim 11, characterized in that the cationic cosurfactant is chosen from the quaternary ammonium salts.

16. Composition according to any one of Claims 1 to 15, characterized in that the cosmetically acceptable medium consists of water or a mixture of water and of a cosmetically acceptable solvent.

17. Composition according to any one of Claims 1 to 16, characterized in that it is provided in the form of a more or less thickened liquid, a gel, an emulsion, an aqueous/alcoholic lotion, a dispersion or an aerosol foam.

18. Composition according to any one of Claims 1 to 17, characterized in that it furthermore contains additives chosen from foam reinforcers, thickeners, sequestering agents, electrolytes, fragrances, preservatives, fatty alcohols, mineral, vegetable or [sic] animal or synthetic oils or waxes, ceramides, UV screening agents, agents for combating free radicals, pearlescence agents, biocides, antibacterials, antidandruff agents, antiseborrheic agents, antiparasitic agents, repellents, dyes, pigments, oxidizing agents, reducing agents, moisturizers, anionic, nonionic or cationic polymers, vitamins or α-hydroxy acids.

19. Use of the composition as defined in any one of Claims 1 to 18 for treating and/or washing keratinous substances consisting of the hair or the skin.

20. Process for cosmetic washing and/or conditioning of the hair or of the skin, characterized in that it consists in applying an effective amount of composition according to any one of Claims 1 to 18 to the skin or the hair, this application optionally being followed by a rinsing with water.

## Patentansprüche

1. Kosmetische Zusammensetzung,
dadurch **gekennzeichnet**, daß
sie in einem wässrigen kosmetisch geeigneten Milieu enthält:
(A) mindestens ein anionisches oberflächenaktives Mittel vom Alxylgalactosiduronat-Typ der Formel: 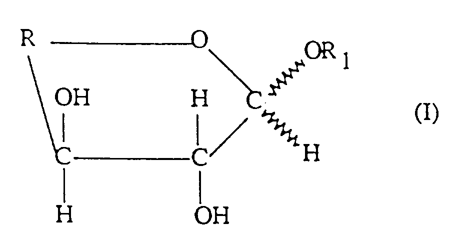 worin gilt:
R₁ bedeutet einen linearen oder verzweigten Alkylrest mit 8 bis 22 Kohlenstoffatomen;
R bedeutet ein Gruppe:
(i) 〉CH -CH(OH) - CO₂R₂ oder
(ii) 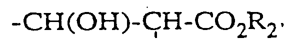 deren die Carboxylgruppe aufweisender Kohlenstoff an das endozyklische Sauerstoffatom gebunden ist, und worin R₂ Wasserstoff, ein Alkalimetall, ein Erdalkalimetall oder eine gegebenenfalls mit Alkyl- oder Hydroxyalkylresten substituierte quaternäre Ammoniumgruppe oder eine von Aminosäuren abgeleitete quaternäre Ammoniumgruppe ist;
(B) mindestens ein amphoteres Polymer.

2. Zusammensetzung gemäß Anspruch 1,
dadurch **gekennzeichnet**, daß
in der Formel (I) der Rest R₂ Natrium oder Kalium, Magnesium oder eine quaternäre Ammoniumgruppe bedeutet, die von Ammoniak, Triethanolamin, Monoethanolamin, 2-Amino-2-methylpropan-1,3-diol, 2-Methyl-2-aminopropan-1-ol, Histidin, Arginin oder Lysin abgeleitet ist.

3. Zusammensetzung gemäß Anspruch 1 oder 2,
dadurch **gekennzeichnet**, daß
die Verbindungen der Formel (I) aus denjenigen ausgewählt sind, für die R₁ einen C₈₋₁₄-Alkylrest bedeutet.

4. Zusammensetzung gemäß einem der Ansprüche 1 bis 3,
dadurch **gekennzeichnet**, daß
die Verbindungen der Formel (I) aus derjenigen ausgewählt ist, für die R₁ den Decylrest bedeutet.

5. Zusammensetzung gemäß Anspruch 4,
dadurch **gekennzeichnet**, daß
die Verbindung der Formel (I) ausgewählt ist aus:
Natrium-Decyl-α-D-galactopyranosiduronat,
Natrium-Decyl-β-D-galactopyranosiduronat,
Natrium-Decyl-α-D-galactofuranosiduronat und aus
Natrium-Decyl-β-D-galactofuranosiduronat.

6. Zusammensetzung gemäß einem der Ansprüche 1 bis 4,
dadurch **gekennzeichnet**, daß
das amphotere Polymer aus Polymeren ausgewählt ist, die Einheiten A und B aufweisen, die statistisch in der Polymerkette verteilt sind, worin
A eine Einheit, die aus einem Monomer abgeleitet ist, das mindestens ein basisches Stickstoffatom aufweist, und B eine Einheit bedeuten, die aus einem Säure-Monomer abgeleitet ist, das eine oder mehrere Carboxyl- oder Sulfonylgruppen aufweist,
oder worin A und B auch Gruppierungen bedeuten können, die aus zwitterionischen Carboxybetain- oder Sulfobetain-Monomeren abgeleitet sind,
wobei A und B auch eine kationische Polymerkette bedeuten können, die primäre, sekundäre, tertiäre oder quaternäre Amingruppen aufweist, worin mindestens eine der Amingruppen eine Carboxyl- oder Sulfonylgruppe aufweist, die über das Zwischenglied eins Kohlenwasserstoffrestes gebunden ist,
oder worin A und B auch Teil einer Polymerkette mit einer α,β-dicarboxylischen Ethylen-Einheit sind, wovon eine der Carboxylgruppen mit einem Polyamin umgesetzt worden ist, das eine oder mehrere primäre oder sekundäre Amingruppen aufweist.

7. Zusammensetzung gemäß Anspruch 6,
dadurch **gekennzeichnet**, daß
die amphoteren Polymeren aus den folgenden Polymeren ausgewählt sind:
(1) aus Polymeren, die sich aus der Copolymerisation eines Monomer, das von einer eine Carboxylgruppe aufweisenden Vinylverbindung abgeleitet ist, wie insbesondere von Acryl-, Methacryl-, Malein- und von α-Chloracrylsäure, und eines basischen Monomer ergeben, das von einer substituierten Vinylverbindung abgeleitet ist, die mindestens ein basisches Stickstoffatom enthält, wie insbesondere Polymere aus Dialkylaminoalkylmethacrylat und Acrylat sowie aus Diaminoalkylmethacrylamid und Acrylamid;
(2) aus Polymeren, die aus einer Diallyldialkylammonium-Verbindung und mindestens einem anionischen Monomer abgeleitet sind, wie Polymere, die ca. 60 bis ca. 99 Gew.% Einheiten, die von einem quaternären Diallyldialkylammonium-Monomer abgeleitet sind, worin die Alkylgruppen, unabhängig voneinander, aus Alkylgruppen mit 1 bis 18 Kohlenstoffatomen ausgewählt und das Anion von einer Säure mit einer Ionisationskonstante von mehr als 10⁻¹³ abgeleitet sind, sowie 1 bis 40 Gew.%, bezogen auf dieses Polymer, eines anionischen Monomer umfassen, das aus Acryl- oder Methacrylsäure ausgewählt ist, wobei das Molekulargewicht dieses Polymer ca. 50 000 bis 10 000 000 beträgt, bestimmt durch Gelpermeationschromatographie;
(3) aus Polymeren, die Einheiten aufweisen, die abgeleitet sind aus:
a) mindestens einem Monomer, ausgewählt aus Acryl- oder Methacrylamiden, welche am Stickstoff mit einem Alkylrest substituiert sind,
b) mindestens einem Säure-Comonomer, das eine oder mehrere reaktive Carboxylgruppen enthält, und
c) mindestens einem basischen Comonomer wie aus Estern mit primären, sekundären, tertiären oder quaternären Aminsubstituenten von Acryl- und Methacrylsäuren sowie aus dem Quaternierungsprodukt von Dimethylaminoethylmethacrylat mit Dimethyl- oder Diethylsulfat;
(4) aus vernetzten und teilweise oder vollständig alkoylierten Polyaminoamiden, abgeleitet aus Polyaminoamiden der allgemeinen Formel:
⁅OC―R₇―CO―Z⁆ (II)
worin R₇ einen zweiwertigen Rest, der von einer gesättigten Dicarboxylsäure, einer aliphatischen Mono- oder Dicarboxylsäure mit ethylenischer Doppelbindung, einem Ester eines 1 bis 6 Kohlenstoffatome aufweisenden Niedrigalkanols dieser Säuren oder einem Rest abgeleitet ist, der wiederum aus der Addition einer der genannten Säuren an ein bisprimäres oder bissekundäres Amin stammt, und Z einen Rest eines bisprimären, mono- oder bissekundären Polyalkylenpolyamins bedeuten, und welcher vorzugsweise darstellt:
a) in Mengenanteilen von 60 bis 100 Mol% den Rest:
― NH⁅(CH₂)ₓ ― NH⁆ₙ (III)
worin x = 2 und n = 2 oder 3 oder auch x = 3 und n = 2, wobei dieser Rest aus Diethylentriamin, Triethylentetraamin oder Dipropylentriamin stammt;
b) in Mengenanteilen von 0 bis 40 Mol% den obigen Rest (III), worin x = 2 und n = 1, welcher aus Ethylendiamin stammt, oder den aus Piperazin abgeleiteten Rest: 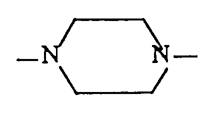
c) in Mengenanteilen von 0 bis 20 Mol% den Rest -NH-(CH₂)₆-NH-, der aus Hexamethylendiamin stammt, wobei diese Polyaminoamide durch Addition eines bifunktionellen Vernetzungsmitttels, das aus Epihalohydrinen, Diepoxiden, Dianhydriden und aus ungesättigten Derivaten ausgewählt ist, mit 0,025 bis 0,35 Mol Vernetzungsmittel pro Amingruppe des Polyaminoamids vernetzt und durch Einwirkung von Acrylsäure, Chloressigsäure oder eines Alkansultons oder von deren Salzen alkoyliert sind;
(5) aus Polymeren, die zwitterionische Einheiten der Formel aufweisen: 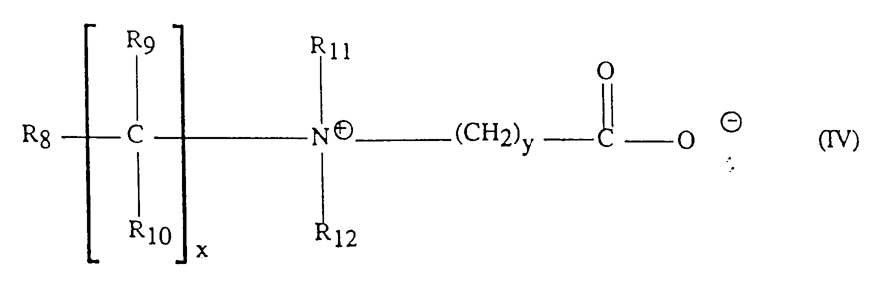 worin R₈ eine ungesättigte polymerisierbare Gruppierung wie eine Acrylat-, Methacrylat-, Acrylamid- oder Methacrylamid-Gruppierung, x und y eine ganze Zahl von 1 bis 3, R₉ und R₁₀ Wasserstoff, einen Methyl-, Ethyl- oder Propylrest, R₁₁ und R₁₂ ein Wasserstoffatom oder einen Alkylrest darstellen, wobei die Summe der Kohlenstoffatome in R₁₁ und R₁₂ 10 nicht übersteigt;
die Polymeren, die derartige Einheiten enthalten, können auch Einheiten aufweisen, die aus nicht-zwitterionischen Monomeren wie aus Vinylpyrrolidon, Dimethyl- oder Diethylaminoethylacrylat oder -methacrylat oder aus Alkylacrylaten oder -methacrylaten, Acrylamiden oder Methacrylamiden oder aus Vinylacetat abgeleitet sind;
(6) aus Polymeren, die aus Chitosan abgeleitet sind, welche monomere Einheiten der folgenden Formeln aufweisen: 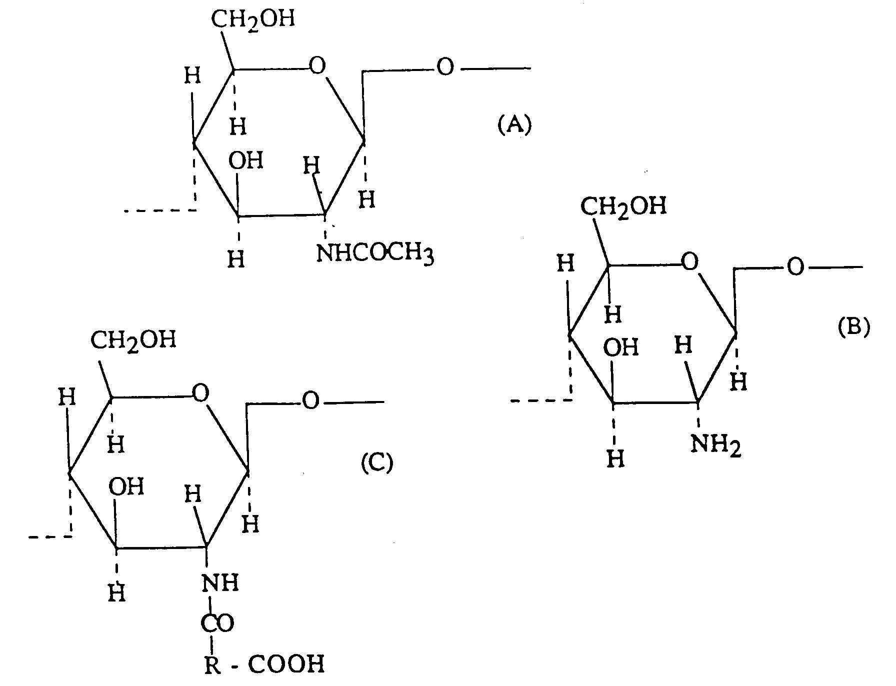 in denen die Einheit A in Mengenanteilen von 0 bis 30%, die Einheit B in Mengenanteilen von 5 bis 50% und die Einheit C in Mengenanteilen von 30 bis 90% vorhanden sind; in der Formel (C) stellt R einen Rest der Formel dar: 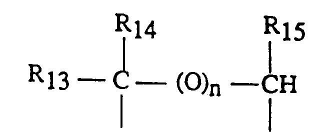 worin, wenn n = 0, R₁₃, R₁₄ und R₁₅, gleich oder verschieden, jeweils ein Wasserstoffatom, einen Methyl-, Hydroxyl-, Acetoxy- oder Aminorest, einen Mono- oder Dialkoylaminrest darstellen, die gegebenenfalls durch ein oder mehrere Stickstoffatome unterbrochen und/oder gegebenenfalls mit einer oder mehreren Amin-, Hydroxy-, Carboxyl-, Alkylthio-, Sulfonylgruppen oder einen Akoylthio-Rest substituiert sind, dessen Alkoylgruppe einen Aminorest aufweist, wobei mindestens einer der Reste R₁₃, R₁₄ und R₁₅ in diesem Fall ein Wasserstoffatom ist, oder worin, wenn n = 1, R₁₃, R₁₄ und R₁₅ jeweils ein Wasserstoffatom darstellen, sowie aus Salzen, die aus diesen Verbindungen mit Basen oder Säuren gebildet sind;
(7) aus Polymeren, die aus einer N-Carboxyalkylierung von Chitosan abgeleitet sind;
(8) aus Polymeren der allgemeinen Formel (V: 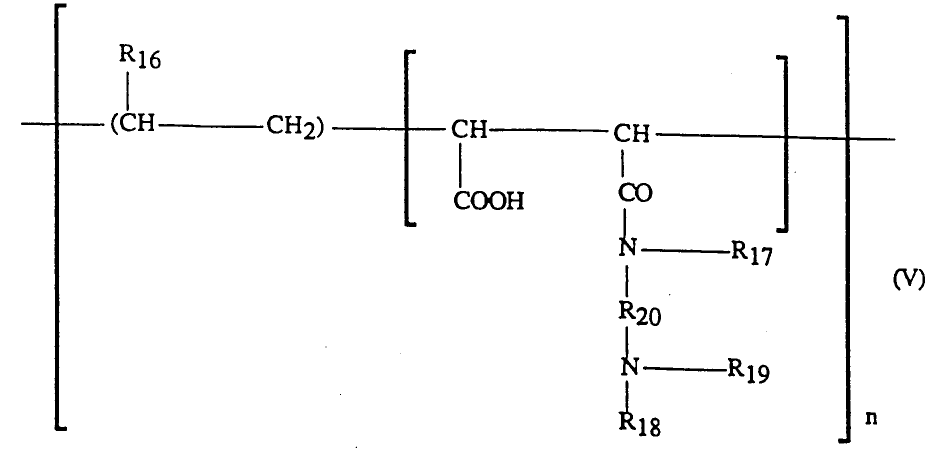 worin R₁₆ ein Wasserstoffatom, einen CH₃O-, CH₃CH₂O- oder einen Phenyl-Rest, R₁₇ Wasserstoff oder einen Niedrigalkylrest wie einen Methyl- oder Ethylrest, R₁₈ Wasserstoff oder einen Niedrigalkylrest wie einen Methyl- oder Ethylrest und R₁₉ einen Niedrigalkylrest wie einen Methyl- oder Ethylrest oder einen Rest der Formel:-R₂₀-N(R₁₈)₂ darstellen, worin R₂₀ eine Gruppierung darstellt: 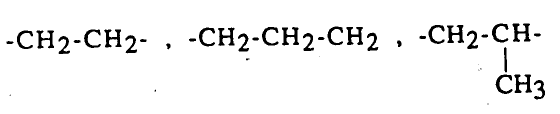 und R₁₈ die oben angegebenen Bedeutungen hat,
wobei auch höhere Homologe dieser Reste eingeschlossen sind, die bis zu 6 Kohlenstoffatome enthalten;
(9) aus amphoteren Polymeren des Typs -A-Z-A-Z, ausgewählt aus:
a) Polymeren, erhältlich durch Einwirkung von Chloressigsäure oder Natriumchloracetat auf Verbindungen mit mindestens einer Einheit der Formel:
-A-Z-A-Z-A- (VI)
worin A einen Rest: 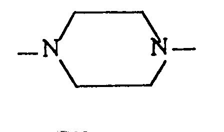 und Z das Symbol B oder B' bedeuten, wobei B oder B', gleich oder verschieden, einen zweiwertigen Rest darstellen, der ein Alkylenrest mit gerader oder verzweigter Kette mit bis zu 7 Kohlenstoffatomen in der Hauptkette ist, die gegebenenfalls mit Hydroxylgruppen substituiert ist und ausserdem Sauerstoff-, Stickstoff- und Schwefelatome und 1 bis 3 aromatische und/oder heterozyklische Ringe aufweisen kann, wobei die Sauerstoff-, Stickstoff- und Schwefelatome in Form einer Ether-, Thioether-, Sulfoxid-, Sulfon-, Sulfonium-, Alkylamin-, Alkenylamin-, Hydroxyl-, Benzylamin-, Aminoxid-, quaternären Ammonium-, Amid-, Imid-, Alkohol-, Ester- und/oder Urethan-Gruppierung vorliegen; und aus
b) Polymeren der Formel -A-Z-A-Z- (VI'), worin A einen Rest: 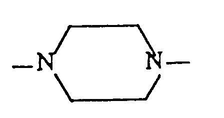 und Z B oder B' und mindestens 1 Mal B' bedeuten, wobei B die oben angegebene Bedeutung hat und B' ein zweiwertiger Rest ist, der ein Alkylenrest mit gerader oder verzweigter Kette mit bis zu 7 Kohlenstoffatomen in der Hauptkette ist, die gegebenenfalls mit einem oder mehreren Hydroxylresten substituiert ist und ein oder mehrere Stickstoffatome aufweist, wobei das Stickstoffatom mit einer gegebenenfalls durch ein Sauerstoffatom unterbrochenen Alkylkette substituiert ist und zwingend eine oder mehrere Carboxyl- oder eine oder mehrere Hydroxyl-Funktionen aufweist, die durch Reaktion mit Chloressigsäure oder Natriumchloracetat in eine Betain-Form überführt sind.

8. Zusammensetzung gemäß einem der Ansprüche 1 bis 7,
dadurch **gekennzeichnet**, daß
die anionischen oberflächenaktiven Mittel der Formel (I) in Mengenanteilen von 1 bis 50 Gew.% und die amphoteren Polieren in Mengenanteilen von 0,1 bis 10 Gew.% vorhanden sind, wobei die Gew.%-Angaben auf das Gesamtgewicht der Zusammensetzung bezogen sind.

9. Zusammensetzung gemäß einem der Ansprüche 1 bis 8 zum Konditionieren keratinischer Materien,
dadurch **gekennzeichnet**, daß
die Konzentration an anionischen oberflächenaktiven Mitteln der Formel (I) 1 bis 10 Gew.% beträgt, bezogen auf das Gesamtgewicht der Zusammensetzung.

10. Zusammensetzung gemäß einem der Ansprüche 1 bis 8 zum Waschen keratinischer Materien,
dadurch **gekennzeichnet**, daß
die Konzentration an anionischen oberflächenaktiven Mitteln in der Formel (I) 4 bis 50 Gew.% beträgt, bezogen auf das Gesamtgewicht der Zusammensetzung.

11. Zusammensetzung gemäß einem der Ansprüche 1 bis 10,
dadurch **gekennzeichnet**, daß
sie zusätzlich ein weiteres oberflächenaktives Co-Mittel vom anionischen, nicht-ionischen, amphoteren, zwitterionischen oder kationischen Typ in einem Mengenanteil bis zu 50% des Gesamtgewichts der in der Zusammensetzung vorhandenen oberflächenaktiven Mittel enthält.

12. Zusammensetzung gemäß Anspruch 11,
dadurch **gekennzeichnet**, daß
das anionische zusätzliche oberflächenaktive Co-Mittel ausgewählt ist aus Alkali-, Ammonium-, Amin-, Aminoalkohol- oder Magnesiumsalzen der folgenden Verbindungen: von Fettsäuren, Alkylsulfaten, Alkylethersulfaten, Alkylamidosulfaten, Alkylarylpolyethersulfaten, Monoglyceridsulfaten, Alkylamidsulfaten, Alkylsulfonaten, Alkylarylsulfonaten, Olefinsulfonaten, Paraffinsulfonaten, Alkylethersulfonaten, Alkylsulfosuccinaten, Alkylethersulfosuccinaten, Alkylamidsulfosuccinaten, Alkylsulfosuccinamaten, Alkylsulfoacetaten, Alkyletherphosphaten, Acylsarcosinaten, N-Acyltauraten, Isethionaten, wobei der Alkyl- oder Acylrest aus einer Kohlenstoffkette mit 10 bis 20 Kohlenstoffatomen oder auch aus polyoxalkylierten Alkylamid- oder Alkylethercarboxylsäuren zusammengesetzt ist.

13. Zusammensetzung gemäß Anspruch 11,
dadurch **gekennzeichnet**, daß
das nicht-ionische oberflächenaktive Co-Mittel aus Alkoholen, α-Diolen oder Alkylphenolen, polyethoxylierten oder polypropoxylierten Fettsäuren mit einer Fettkette mit 8 bis 18 Kohlenstoffatomen, wobei die Anzahl an Ethylen- oder Propylenoxidgruppen 2 bis 50 und die Anzahl an Glycerylgruppen 2 bis 30 betragen, aus Copolymeren aus Ethylen- und Propylenoxiden, Kondensaten von Ethylen- und Propylenoxiden mit Fettalkoholen, polyethoxylierten Fettamiden, polyethoxylierten Fettaminen, oxethylierten Sorbitanfettsäureestern, Zuckerfettsäureestern, Polyethylenglycolfettsäureestern, Fettsäureestern von Glycolen und aus Aminoxiden ausgewählt ist.

14. Zusammensetzung gemäß Anspruch 11,
dadurch **gekennzeichnet**, daß
das zusätzliche amphotere oder zwitterionische oberflächenaktive Co-Mittel aus sekundären oder tertiären aliphatischen Aminderivaten, in denen der aliphatische Rest eine lineare oder verzweigte Kette mit 8 bis 18 Kohlenstoffatomen ist und mindestens eine anionische, Wasserlöslichkeit vermittelnde Carboxylat-, Sulfonat-, Sulfat-, Phosphat- oder Phosphonatgruppe enthält, aus C₈₋₂₀-Alkylbetainen, Sulfobetainen, C₈₋₂₀-Alkylamido-C₁₋₆-alkylbetainen oder C₈₋₂₀-Alkylamido-C₁₋₆-alkylsulfobetainen, Alkylpeptiden und aus Alkylimidazoliumbetainen ausgewählt ist.

15. Zusammensetzung gemäß Anspruch 11,
dadurch **gekennzeichnet**, daß
das zusätzliche kationische oberflächenaktive Co-Mittel aus quaternären Ammoniumsalzen ausgewählt ist.

16. Zusammensetzung gemäß einem der Ansprüche 1 bis 15,
dadurch **gekennzeichnet**, daß
das kosmetisch geeignete Milieu aus Wasser oder aus einer Mischung aus Wasser und einem kosmetisch geeigneten Lösungsmitel zusammengesetzt ist.

17. Zusammensetzung gemäß einem der Ansprüche 1 bis 16,
dadurch **gekennzeichnet**, daß
sie in Form einer mehr oder weniger verdickten Flüssigkeit, eines Gels, einer Emulsion, einer hydroalkoholischen Lotion, einer Dispersion oder eines Aerosol-Schaums vorliegt.

18. Zusammensetzung gemäß einem der Ansprüche 1 bis 17,
dadurch **gekennzeichnet**, daß
sie zusätzlich Additive enthält, ausgewählt aus Schaumverstärkungsmitteln, Verdickungsmitteln, Sequestriermitteln, Elektrolyten, Parfüm-Produkten, Konservierungsmitteln, Fettalkoholen, mineralischen, pflanzlichen oder tierischen Ölen, mineralischen, pflanzlichen, tierischen oder synthetischen Wachsen, Ceramiden, UV-Filterstoffen, Perlmuttglanzmitteln, Abfangmitteln für freie Radikale, Biociden, antibakteriellen Mitteln, Mitteln gegen Haarausfall, antiseborrheischen Mitteln, Mitteln gegen Parasiten, Abschreckmitteln, Farbstoffen, Pigmenten, Oxidations-, Reduktionsmitteln, Hydratisiermitteln, anionischen, nicht-ionischen oder kationischen Polymeren, Vitaminen oder aus α-Hydroxysäuren.

19. Verwendung der in einem der Ansprüche 1 bis 18 definierten Zusammensetzungen zum Behandeln und/oder Waschen keratinischer Materien aus Haaren oder der Haut.

20. Verfahren zum Waschen und/oder kosmetischen Konditionieren der Haare oder der Haut,
dadurch **gekennzeichnet**, daß
man auf die Haut oder die Haare eine ausreichende Menge einer Zusammensetzung gemäß einem der Ansprüche 1 bis 18 aufbringt, worauf auf diese Aufbringung gegebenenfalls eine Spülung mit Wasser erfolgt.
